# EUROPÄISCHE PATENTSCHRIFT

(11) **EP 3 296 388 B1**
(45) Veröffentlichungstag und Bekanntmachung des Hinweises auf die Patenterteilung: **21.10.2026**
(21) Anmeldenummer: 17190041.8
(22) Anmeldetag: 08.09.2017
(51) Int. Cl.: C12M 3/06, C12M 1/00, C12M 1/42, C12M 1/34

(54) **MIKROFLUIDISCHE VORRICHTUNG FÜR ZELLKULTUREXPERIMENTE UND VERWENDUNGEN HIERVON**
MICROFLUIDIC DEVICE FOR CELL CULTURE EXPERIMENTS AND USES THEREOF
DISPOSITIF MICROFLUIDIQUE POUR ESSAIS DE CULTURE CELLULAIRE ET SES UTILISATIONS

(30) Priorität: 09.09.2016 DE 102016217250
(43) Veröffentlichungstag der Anmeldung: 21.03.2018
(73) Patentinhaber: Fraunhofer-Gesellschaft zur Förderung der angewandten Forschung e.V., 80686 München (DE); Technische Universität Dresden, 01062 Dresden (DE)
(72) Erfinder: Schmieder, Florian, 01069 Dresden (DE); Sonntag, Dr. Frank, 01277 Dresden (DE); Klotzbach, Dr. Udo, 01326 Dresden (DE); Hohenstein, Prof. Dr. Bernd, 01445 Radebeul (DE); Hugo, Prof. Dr. Christian, 01445 Radebeul (DE)
(74) Vertreter: Pfenning, Meinig & Partner mbB

(56) Entgegenhaltungen:
- WO-A1-2015/092141
- DE-A1- 10 018 255
- DE-A1- 10 224 725
- DE-A1- 102007 038 777
- DE-A1- 102008 018 170
- DE-A1- 102011 112 638
- DE-A1- 102013 109 481
- DE-B3- 102015 204 728
- WÄLDCHEN, S.: "Light-induced cell damage in live- cell super-resolution microscopy", SCIENTIFIC REPORTS, 2015, XP002777868
- GALBRAITH, C. G. ET AL.: "Super-resolution microscopy at a glance", JOURNAL OF CELL SCIENCE, vol. 124, no. 10, 2011, XP002777869
- FELLER, K. H. ET AL.: "Stresstest für die Haut - Labor der Zukunft - Lab-on-a- chip", LABORPRAXIS, March 2016 (2016-03-01), pages 20 - 22, XP002777870

## Beschreibung

Es wird eine mikrofluidische Vorrichtung für Zellkulturexperimente vorgestellt. Die mikrofluidische Vorrichtung enthält neben mindestens einem Zellgefäß zur Kultivierung von biologischen Zellen eine Vorrichtung zur Bewegung einer Flüssigkeit in dem Zellgefäß, ein Gerät zur Schädigung biologischer Zellen über elektromagnetische Strahlung, eine Steuereinheit zur Steuerung dieses Geräts und eine Vorrichtung zur Untersuchung der biologischen Zellen in dem Zellgefäß, wobei die Vorrichtung dazu geeignet ist, von biologischen Zellen aus dem Zellgefäß emittierte elektromagnetische Strahlung zu detektieren. Die vorgestellte mikrofluidische Vorrichtung hat den Vorteil, dass eine Schädigung gezielt in eine sehr geringe Menge biologischer Zellen eingeführt werden kann, wobei die Schädigung und deren Heilung anschließend auf molekularer Ebene ortsspezifisch und reproduzierbar verfolgt werden kann. Es wird daher vorgeschlagen, die mikrofluidische Vorrichtung zur Untersuchung der Schädigung, Regeneration und Interaktion von biologischen Zellen zu verwenden.

Für wissenschaftliche Untersuchungen der Regenerationsmechanismen sowie der Interaktion verschiedener oder gleichartiger Zellen (z.B. Endothelzellen mit Leukozyten und/oder Thrombozyten) in Zellkultur ist es notwendig, die in einem Zellkulturgefäß kultivierten Zellen - möglichst bis auf die Stufe einer einzelnen Zelle - auf präzise Art und Weise gezielt und ortsselektiv schädigen zu können. Das Problem dabei ist es, dass die Zellkulturgefäße regelmäßig geschlossene und schwer zugängliche Gefäße darstellen, die es erschweren, eine definierte und möglichst partielle Schädigung der kultivierten Zellen vorzunehmen und die Schädigung, sowie die anschließenden Regenerationsmechanismen, sinnvoll (z.B. über Bilder und/oder Videos) auszuwerten und zu dokumentieren.

Verfahren zur Schädigung von Zellen in Zellkulturgefäßen sind im Stand der Technik bekannt. Als einfach zu nutzendes wissenschaftliches Standardverfahren sind sog. "Anritz-Assays" (engl. "scratch-assays") weit verbreitet. Diese können einfach durchgeführt werden, indem eine konfluente Zellschicht mit einer Pipettenspitze angeritzt wird und die Regeneration der angeritzten Zellschicht unter dem Mikroskop verfolgt wird (z .B. über Zeitablauf-Aufnahmen).

Zudem ist im Stand der Technik eine Vorrichtung bekannt, in der Zellen auf der Innenoberfläche eines Kanals kultiviert werden, der von Zellkulturmedium durchströmt wird. Durch das halbseitige Durchspülen des Kanals mit einer Lösung des Enzyms Trypsin wird die Hälfte der Zellen von der Innenoberfläche des Kanals abgelöst und aus dem Kanal ausgewaschen, was zu einer großflächigen Schädigung der Zellschicht führt. Diese Vorrichtung wird anschließend auf dem Plattenteller eines Mikroskops montiert und die Schädigung und auch Regeneration der Zellen kann optisch detektiert und verfolgt werden.

In dieser Hinsicht ist zudem bekannt, in einer Mikrotiterplatte anstelle der Ablösung der Zellen durch Trypsin zunächst eine bestimmte Fläche auf der Mikrotiterplatte zu maskieren und anschließend die Maskierung zu lösen, um das Wachstum der Zellen bis zur Konfluenz zu verfolgen. Zudem ist es bekannt, Zellen gezielt durch Kontaktieren der Zellen mit bestimmten chemischen Stoffen zu schädigen und anschließend die Regeneration der geschädigten Zellen zu untersuchen und zu verfolgen.

Der entscheidende Nachteil der im Stand der Technik bekannten Vorrichtungen und Verfahren liegt in der Erzeugung einer verhältnismäßig großen Wundfläche (mehr als 100 geschädigte Zellen), wodurch viele Schädigungen im Körper nur unzureichend abgebildet werden können.

Ferner wird bei den im Stand der Technik bekannten Verfahren teilweise lediglich eine mit Zellen bedeckte Fläche maskiert d.h. keine wirkliche Schädigung der Zellen durchgeführt.

Zudem sind bei den Vorrichtungen und Verfahren im Stand der Technik die Möglichkeit zur optischen Untersuchung und Verfolgung der Schädigung und Regeneration wenig reproduzierbar und sehr begrenzt. Tatsächlich beschränkt sich die optische Auswertung mit Vorrichtungen aus dem Stand der Technik auf Informationen, die mit gängigen optischen Mikroskopen erfasst werden können. Eine Auswertung eines breiten Spektrums der elektromagnetischen Strahlung bzw. ausgewählter Wellenlängen ist bislang nicht möglich. Damit sind nennenswerte Informationen dieser Assays bislang nicht zugänglich.

Es sind Verfahren im Stand der Technik bekannt, um Zellen gezielt aus einem Gewebe auszuschneiden (z.B. die sog. Lasermikrodissektion; siehe DE 100 18 255 A1). Ferner ist im Stand der Technik bekannt, einen IR-Laser zu verwenden, um Wasserdampf zu erzeugen und eine Zellschädigung von Zellen in einer Zellkultur herbeizuführen (siehe DE 10 2013 109 481 A3). Darüberhinaus ist im Stand der Technik eine mikrofluidische Vorrichtung zum Bestimmen einer Anzahl biologischer Zellen bekannt, die eine elektromagnetische Strahlungsquelle enthält und eine Detektionseinheit zur Erfassung elektromagnetischer Strahlung aufweist (siehe DE 10 2015 204 728 B3). Wäldchen, S. et al. (Scientific Reports, 2015, 5:15348, p. 1-12) lehren Strategien zur Verringerung einer lichtbedingten Schädigung, die bei einer Bestrahlung von biologischen Zellen mit elektromagnetischer Strahlung kurzer Wellenlänge und hoher Lichtintensität auftreten kann. Die DE 10 2011 112 638 A1 offenbart einen mikrofluidischen Chip, mit dem kleine Blutgefäße unter Flussbedingungen simuliert werden können. Die DE 102 24 725 A1 offenbart ein Mikrofluidsystem, mit dem auch ohne externen Anschluss in einfacher Weise Flüssigkeiten transportiert werden können.

Ausgehend hiervon war es die Aufgabe der vorliegenden Erfindung ein mikrofluidisches System bereitzustellen, das es erlaubt, eine Schädigung auf einer geringen Fläche, d.h. bei einer geringen Anzahl, biologischer Zellen zu bewirken und die Schädigung und/oder die Heilung der Zellen auf molekularer Ebene ortsspezifisch und reproduzierbar verfolgen zu können.

Die Aufgabe wird gelöst durch das mikrofluidische System mit den Merkmalen von Anspruch 1 und die Verwendung des mikrofluidischen Systems gemäß Anspruch 15. Die abhängigen Ansprüche zeigen vorteilhafte Weiterbildungen auf.

Erfindungsgemäß wird eine Vorrichtung für Zellkulturexperimente bereitgestellt, enthaltend
a) mindestens ein Zellgefäß zur Kultivierung von biologischen Zellen;
b) ein Gerät zur Schädigung biologischer Zellen, wobei das Gerät dazu geeignet ist, elektromagnetische Strahlung auf biologische Zellen in dem Zellgefäß zu emittieren;
c) eine Steuereinheit zur Steuerung des Geräts zur Schädigung biologischer Zellen in dem Zellgefäß; und
d) eine Vorrichtung zur Untersuchung der biologischen Zellen in dem Zellgefäß, wobei die Vorrichtung dazu geeignet ist, von biologischen Zellen aus dem Zellgefäß emittierte elektromagnetische Strahlung zu detektieren;
dadurch gekennzeichnet, dass
die Vorrichtung eine mikrofluidische Vorrichtung ist,
die mikrofluidische Vorrichtung eine Mikropumpe zur Bewegung einer Flüssigkeit in dem Zellgefäß enthält,
die mikrofluidische Vorrichtung aus mehreren, übereinander angeordneten Schichten aufgebaut ist, wobei mindestens eine Schicht ein pneumatisches Subsystem aufweist, das mindestens einen Kanal zum Transport eines Gases enthält,
das Zellgefäß in einem fluidischen Kreislauf angeordnet ist, und
die mikrofluidische Vorrichtung eine Systemsteuervorrichtung enthält, wobei die Systemsteuervorrichtung dazu konfiguriert ist, die Steuereinheit zur Steuerung des Geräts zur Schädigung biologischer Zellen, die Vorrichtung zur Untersuchung der biologischen Zellen und die Mikropumpe zur Bewegung einer Flüssigkeit zu steuern.

Unter dem Begriff "mikrofluidisch" wird verstanden, dass (alle) Fluid-leitende (z.B. Flüssigkeits-leitenden) Komponenten der Vorrichtung (z.B. Zellgefäß und/oder die fluidleitenden Komponenten der Vorrichtung zur Bewegung der Flüssigkeit in dem Zellgefäß) bevorzugt einen Maßstab im Mikrometerbereich aufweisen. In dieser Hinsicht ist es bevorzugt, dass das Zellgefäß eine Länge, Breite und/oder Höhe aufweist, die 1000 µm nicht überschreitet, besonders bevorzugt eine Länge, Breite und/oder Höhe, die im Bereich von 1 µm bis 1000 µm angesiedelt ist, insbesondere eine Länge, Breite und Höhe, die im Bereich von 1 µm bis 1000 µm angesiedelt ist.

Der wichtigste Vorteil der hier vorgestellten Entwicklung ist die Möglichkeit, über eine elektromagnetische Strahlungsquelle (z.B. ein optisches System) definiert Zellschädigungen von beliebig vielen Zellen bis hin zur Einzelzelle in geschlossenen oder halboffenen Zellkultursystemen vorzunehmen. Ein weiterer Vorteil ist, dass es die Vorrichtung zur Untersuchung biologischer Zellen als Teil der mikrofluidischen Vorrichtung ermöglicht, auf schnelle, einfache und reproduzierbare Art und Weise eine ortsspezifische Schädigung der Zellen, sowie die anschließende Regeneration dieser Schädigung, bis auf die molekulare Ebene zu verfolgen.

Die mikrofluidische Vorrichtung kann dadurch gekennzeichnet sein, dass die mikrofluidische Vorrichtung, bevorzugt das Zellgefäß zur Kultivierung biologischer Zellen
i) mindestens eine Kammer aufweist, wobei die mindestens eine Kammer bevorzugt eine Länge, Breite und/oder Höhe unter 1000 µm, besonders bevorzugt eine Länge, Breite und/oder Höhe von 1 µm bis 1000 µm, insbesondere eine Länge, Breite und Höhe im Bereich von 1 µm bis 1000 µm hat; und/oder
ii) mindestens einen Kanal aufweist, wobei der mindestens eine Kanal senkrecht zur Ausbreitungsrichtung des Kanals, bevorzugt eine Ausdehnung von maximal 1000 µm, besonders bevorzugt eine Ausdehnung von 1 µm bis 1000 µm hat; und/oder
iii) mindestens eine Oberfläche mit einer Markierung aufweist, bevorzugt mindestens eine Oberfläche mit einem Gitter, besonders bevorzugt mindestens eine Oberfläche mit einem mit Koordinaten versehenen Gitter, wobei die Oberfläche insbesondere eine Bodenfläche des Zellgefäßes ist; und/oder
iv) fluidisch mit einer Mikropumpe verbunden ist, wobei die Mikropumpe bevorzugt mindestens ein Einlassventil, mindestens eine Pumpkammer und mindestens ein Auslassventil enthält oder daraus besteht; und/oder
v) symmetrisch aufgebaut ist, wobei die mikrofluidische Vorrichtung bevorzugt mindestens zwei symmetrisch angeordnete fluidische Kreisläufe enthält.

Optional kann die mindestens eine Kammer mit dem mindestens einen Kanal fluidisch verbunden sein. Das Zellgefäß ist erfindungsgemäß in einem fluidischen Kreislauf angeordnet, der optional mindestens ein weiteres Zellgefäß und mindestens einen Kanal, bevorzugt mehrere weitere Zellgefäße und mehrere Kanäle, enthält.

Die mikrofluidische Vorrichtung kann
i) ein pneumatisches Subsystem aufweisen, das mehrere Kanäle zum Transport eines Gases enthält; und/oder
ii) mindestens eine selektiv permeable Schicht enthalten, die für Gas, bevorzugt für Sauerstoff und/oder Kohlendioxid, permeabel ist und/oder für Flüssigkeit impermeabel ist, wobei die selektiv permeable Schicht bevorzugt eine elastomere Membran enthält oder daraus besteht; und/oder
iii) mindestens eine Schicht, bevorzugt mehrere Schichten, ein fluidisches Subsystem aufweisen, wobei das fluidische Subsystem insbesondere mindestens eine Schicht mit mindestens einer Mikropumpe, mindestens eine Schicht mit mindestens einem Zellgefäß, mindestens eine Schicht mit Kanälen und/oder mindestens eine Schicht mit Markierungen enthält;
dadurch gekennzeichnet, dass mindestens eine Schicht, bevorzugt alle Schichten für elektromagnetische Strahlung im VIS-Bereich, bevorzugt im UV-Bereich und VIS-Bereich, besonders bevorzugt im UV-Bereich, VIS-Bereich und IR-Bereich transparent ist/sind.

Ferner kann die mikrofluidische Vorrichtung, bevorzugt das Zellgefäß zur Kultivierung von biologischen Zellen, optional mindestens eine Kammer und/oder mindestens ein Kanal, eine Membran aufweisen, die zur Aufwachsung biologischer Zellen geeignet ist.

Die mikrofluidische Vorrichtung, bevorzugt das Zellgefäß zur Kultivierung biologischer Zellen, kann eine Quelle ausgewählt aus der Gruppe bestehend aus Heizquelle, Kühlquelle, Gasquelle, Flüssigkeitsquelle und Kombinationen hiervon, bevorzugt eine Quelle ausgewählt aus der Gruppe bestehend aus Heizquelle zur Temperierung auf 25 bis 37 °C, Sauerstoffquelle, Kohlendioxidquelle, Wasserdampfquelle und Kombinationen hiervon, enthalten.

In einer bevorzugten Ausgestaltungsform enthält die mikrofluidische Vorrichtung, bevorzugt das Zellgefäß zur Kultivierung biologischer Zellen, ein Material ausgewählt aus der Gruppe bestehend aus Polymere, bevorzugt ein Material ausgewählt aus der Gruppe bestehend aus Polycarbonat, Cyclo-Olefin-Copolymere, transparente Elastomere, Glas und Mischungen hiervon, besonders ein Material ausgewählt aus der Gruppe bestehend aus PDMS, Topas^{®}-Cyclo-Olefin-Copolymer und Mischungen hiervon, oder besteht daraus.

Die Mikropumpe kann mindestens ein Einlassventil, mindestens eine Pumpkammer und mindestens ein Auslassventil enthalten oder daraus bestehen.

In einer bevorzugten Ausgestaltungsform enthält die mikrofluidische Vorrichtung, bevorzugt das Gerät zur Schädigung biologischer Zellen, eine elektromagnetische Strahlungsquelle, bevorzugt eine elektromagnetische Strahlungsquelle, die dazu geeignet ist
i) elektromagnetische Strahlung im UV-Bereich und/oder VIS-Bereich, abzustrahlen; und/oder
ii) kohärente und/oder monochromatische elektromagnetische Strahlung abzustrahlen, wobei das Gerät bevorzugt einen Laser enthält oder daraus besteht.

Die mikrofluidische Vorrichtung kann eine Schneidevorrichtung enthalten, bevorzugt eine Schneidevorrichtung zum Schneiden von dem Zellgefäß zur Kultivierung biologischer Zellen, besonders bevorzugt eine Schneidvorrichtung zum Schneiden von einer Kammer, einem Kanal und/oder einer Zellwand der mikrofluidischen Vorrichtung, wobei die Schneidevorrichtung insbesondere eine mechanische Schneidvorrichtung, bevorzugt ein Messer, und/oder eine elektromagnetische Strahlungsquelle enthält oder daraus besteht.

Die Steuervorrichtung kann dazu konfiguriert sein, das Gerät zur Schädigung biologischer Zellen über eine Steuergröße ausgewählt aus der Gruppe bestehend aus Bestrahlungsposition entlang des Zellgefäßes, Bestrahlungsbeginn, Bestrahlungsende, Bestrahlungszeit und Bestrahlungsintensität zu steuern.

Die mikrofluische Vorrichtung, bevorzugt das Gerät zur Schädigung biologischer Zellen und/oder die Vorrichtung zur Untersuchung biologischer Zellen, kann
i) eine optische Strahlenführung zur Leitung elektromagnetischer Strahlung, bevorzugt eine optische Strahlenführung ausgewählt aus der Gruppe bestehend aus optische Faser, optischer Wellenleiter, digitaler Mikrospiegel und Kombinationen hiervon; und/oder
ii) eine Vorrichtung zur Fokussierung elektromagnetischer Strahlung, bevorzugt eine Vorrichtung ausgewählt aus der Gruppe bestehend aus Linse, Spiegel und Kombinationen hiervon; und/oder
iii) eine Vorrichtung zur Filterung elektromagnetischer Strahlung, bevorzugt ausgewählt aus der Gruppe bestehend aus optischer Filter, digitaler Mikrospiegel und Kombinationen hiervor, besonders bevorzugt einen optischen, dichroitischen Filter, einen digitalen Mikrospiegel und Kombinationen hiervon;
enthalten. Der Vorteil an einem digitalen Mikrospiegel zur Strahlungsführung ist, dass kein bewegbarer x,y,z-Tisch nötig ist, mit dem das Zellgefäß zur Kultivierung von biologischen Zellen relativ zur Strahlungsquelle in eine x-Richtung, y-Richtung bzw. eine z-Richtung bewegt werden kann. Mit anderen Worten ist die ortsspezifische Bestrahlung hier allein über den digitalen Mikrospiegel möglich.

Die mikrofluidische Vorrichtung, bevorzugt die Vorrichtung zur Untersuchung biologischer Zellen, kann ein optisches Mikroskop, bevorzugt ein optisches und elektronisches Mikroskop, enthalten oder daraus bestehen.

Die mikrofluidische Vorrichtung, bevorzugt die Vorrichtung zur Untersuchung biologischer Zellen, kann eine
i) elektromagnetische Strahlungsquelle, bevorzugt eine elektromagnetische Strahlungsquelle mit homogener Intensitätsverteilung, wobei die Strahlungsquelle insbesondere die dazu geeignet ist, elektromagnetische Strahlung im UV-Bereich und/oder VIS-Bereich abzustrahlen; und/oder
ii) eine Detektionsvorrichtung zur Detektion von elektromagnetischer Strahlung, bevorzugt einen HSI-Detektor, besonders bevorzugt einen HSI-Detektor zur Detektion von elektromagnetischer Strahlung im UV-Bereich, VIS-Bereich, IR-Bereich und/oder FIR-Bereich, insbesondere einen HSI-Detektor zur Detektion von elektromagnetischer Strahlung im UV-Bereich, VIS-Bereich, IR-Bereich und FIR-Bereich;
enthalten.

Die mikrofluidische Vorrichtung, bevorzugt die Vorrichtung zur Untersuchung biologischer Zellen, kann eine Aufzeichnungsvorrichtung, bevorzugt eine Kamera, besonders bevorzugt einer Kamera zur Detektion von elektromagnetischer Strahlung im Nah-IR und/oder VIS-Bereich, besonders bevorzugt eine CCD-Kamera, enthalten.

Die mikrofluidische Vorrichtung, bevorzugt die Vorrichtung zur Untersuchung biologischer Zellen und/oder das Gerät zur Schädigung biologischer Zellen, kann ein Ortungssystem, bevorzugt ein Ortungssystem zur Verfolgung von Zellen, besonders bevorzugt ein Ortungssystem zur Verfolgung einzelner Zellen, insbesondere eine Kombination aus Kamera und Bildauswertungsprogramm, enthalten.

In einer bevorzugten Ausgestaltungsform ist die Vorrichtung zur Untersuchung biologischer Zellen optisch und/oder elektronisch an das Gerät zur Schädigung biologischer Zellen gekoppelt, wobei das Gerät zur Schädigung biologischer Zellen und die Vorrichtung zur Untersuchung biologischer Zellen optional als eine zusammenhängende Einheit ausgestaltet sind.

Die mikrofluidische Vorrichtung enthält erfindungsgemäß eine Systemsteuervorrichtung, wobei die Systemsteuervorrichtung dazu konfiguriert ist, die Steuereinheit zur Steuerung des Geräts zur Schädigung biologischer Zellen, die Vorrichtung zur Untersuchung der biologischen Zellen und die Vorrichtung zur Bewegung einer Flüssigkeit zu steuern, optional zudem dazu konfiguriert ist, eine zusätzliche Lichtquelle zu steuern.

Ferner wird die Verwendung einer erfindungsgemäßen mikrofluidischen Vorrichtung zur
i) Untersuchung einer Schädigung von biologischen Zellen, bevorzugt zur Schädigung der zellulären Membran von biologischen Zellen, besonders bevorzugt zur Untersuchung der Wirkung von mindestens einem Medikament auf die Schädigung der zellulären Membran von biologischen Zellen; und/oder
ii) Untersuchung einer Regeneration von biologischen Zellen, bevorzugt zur Untersuchung der Regeneration von biologischen Zellen nach einer Schädigung, besonders bevorzugt zur Untersuchung der Wirkung von mindestens einem Medikament auf die Regeneration von biologischen Zellen nach einer Schädigung; und/oder
iii) Untersuchung der räumlichen Interaktion verschiedener Zelltypen, bevorzugt der räumlichen Interaktion von immunologischen Zelltypen mit Endothelzellen;
vorgeschlagen. Insbesondere kann die Untersuchung der räumlichen Interaktion von autoimmunologischen Zellen und Endothelzellen wichtige Informationen über den Verlauf entzündlicher Erkrankungen liefern.

Anhand der nachfolgenden Figuren soll der erfindungsgemäße Gegenstand näher beschrieben werden ohne diesen auf die hier dargestellten, spezifischen Ausgestaltungsformen einschränken zu wollen.

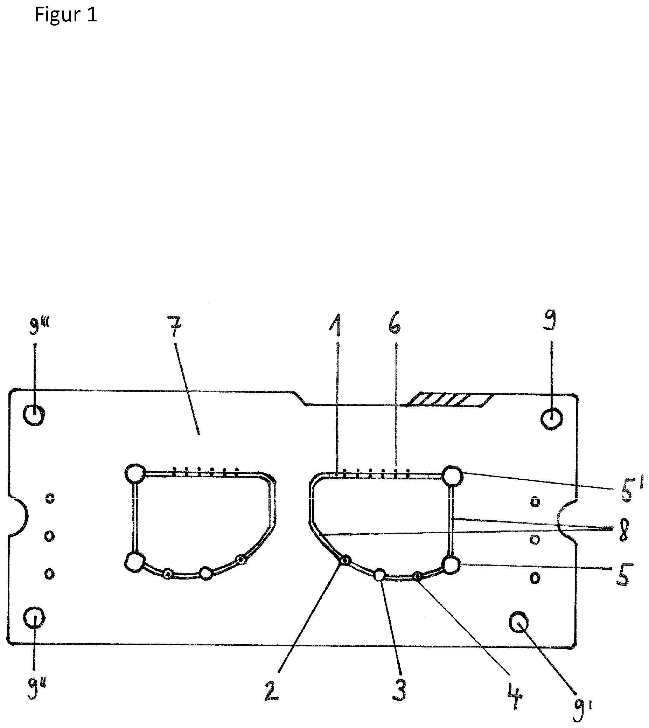

Figur 1 zeigt die Ausgestaltung eines Zellgefäßes 1, hier vielmehr zwei Zellgefäße 1, wie es/sie in der erfindungsgemäßen Vorrichtung vorkommen kann. In dieser Ausgestaltungsform sind zwei Zellgefäße 1 jeweils einer mikrofluidischen Anordnung auf einem Mikrofluidikchip 7 angeordnet, wobei die beiden mikrofluidischen Anordnungen symmetrisch zueinander auf dem Mikrofluidikkchip 7 angeordnet sind. Die beiden Anordnungen weisen jeweils ein kanalförmiges Zellgefäß 1 auf, das sich mit einem Einlassventil 2, einer Pumpenkammer 3, einem Auslassventil 4, einem ersten Reservoir 5 und einem zweiten Reservoir 5' in einem fluidischen Kreislauf befindet, wobei die einzelnen Komponenten über einen Kanal 8 miteinander verbunden sind. Am Boden des Zellgefäßes 1 und hier auch an dessen Seite befinden sich Markierungen 6. Das Einlassventil 2, die Pumpenkammer 3 und das Auslassventil 4 funktionieren zusammen als Mikropumpe. Die Mikropumpe kann mindestens eine Elastomer-Membran aufweisen und somit gleichzeitig als Gasaustauscher und/oder Gasquelle (Sauerstoff und/oder Kohlendioxid) dienen. In dieser Ausgestaltungsform weist der Mikrofluidikchip 7 ferner Aussparungen 9, 9', 9", 9‴ zur stabilen Befestigung in der erfindungsgemäßen Vorrichtung auf.

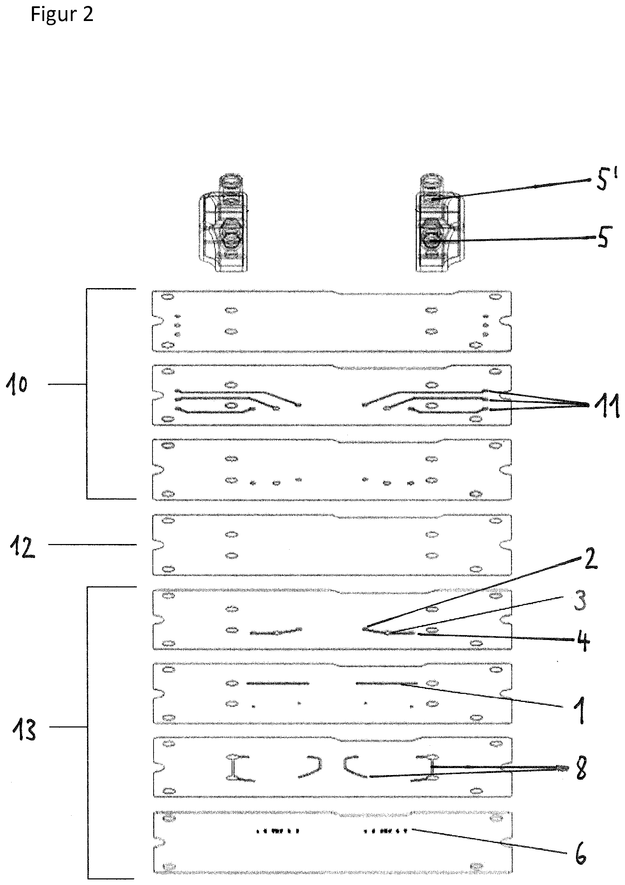

Figur 2 zeigt eine Ausgestaltungsform eines Mikrochips 7 mit Zellgefäß 1, wobei der Aufbau des Mikrochips 7 hier in einzelnen Schichten erfolgt, die jeweils dargestellt sind. Die obersten drei Schichten 10 bauen zusammen ein pneumatisches Subsystem auf, das Gaskanäle 11 zur Zuführung und/oder Abführung von Gas aufweist. Auf der obersten Schicht der obersten drei Schichten 10 sind jeweils symmetrisch das erste Reservoir 5 und das zweite Reservoir 5' der beiden symmetrischen fluidischen Kreisläufe angeordnet. Unter den drei oberen Schichten 10 ist eine elastomere Membran 12 angeordnet, wobei die elastomere Membran gaspermeabel ausgestaltet ist d.h. eine Gasdiffusion aus dem pneumatischen Subsystem heraus und in das pneumatische Subsystem hinein erlaubt. Unter der elastomeren Membran sind die untersten vier Schichten 13 angeordnet, die zusammen ein fluidisches Subsystem aufbauen. Die erste, an die elastomere Membran 12 angrenzende Schicht enthält symmetrisch angeordnet jeweils eine Mikropumpe, d.h. symmetrisch angeordnet jeweils ein Einlassventil 2, eine Pumpenkammer 3 und ein Auslassventil 4. Die nach unten angrenzende Schicht des fluidischen Subsystems weist symmetrisch angeordnet jeweils ein Zellgefäß 1 auf. Es folgt die dritte Schicht, in der die Kanäle 8 angeordnet sind, welche das Zellgefäß 1 mit der Mikropumpe fluidisch verbinden. Auf der untersten, vierten Schicht des Mikrochips 7 sind Markierungen 6 angebracht, die im zusammengesetzten Mikrochip 7 senkrecht unter und/oder leicht versetzt neben dem Zellgefäß 1 zum Liegen kommen. Alle in dieser Ausgestaltungsform dargestellten Schichten sind zumindest für sichtbares Licht transparent.

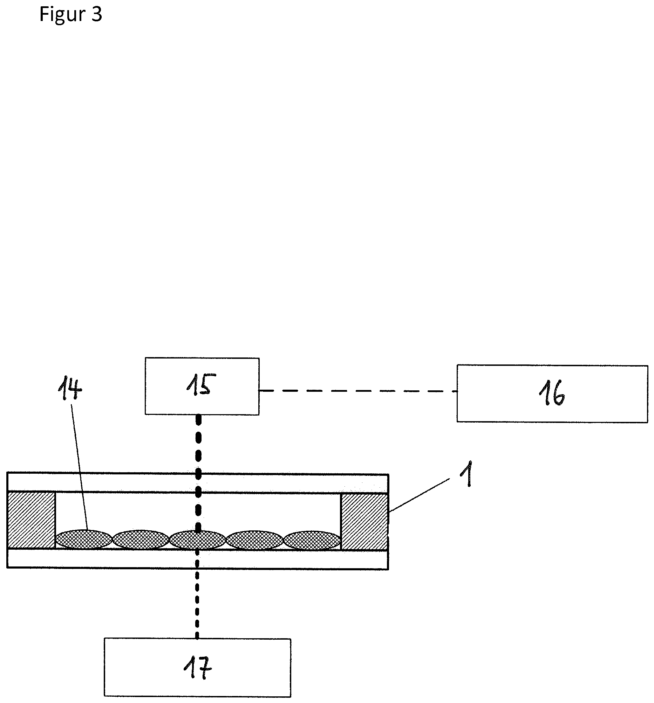

Figur 3 zeigt eine Ausgestaltungsform einer erfindungsgemäßen Vorrichtung. In dem Zellgefäß 1 zur Kultivierung von biologischen Zellen sind hier am Boden des Zellgefäßes 1 die biologischen Zellen 14 dargestellt. Die Vorrichtung zur Bewegung einer Flüssigkeit in dem Zellgefäß, beispielsweise eine Mikropumpe, ist hier nicht dargestellt. Das Gerät 15 zur Schädigung biologischer Zellen ist in dieser Ausgestaltungsform ein Laser. Der Laser 15 ist kommunikativ mit einer Steuereinheit 16 zur Steuerung des Lasers verbunden. Die Vorrichtung 17 zur Untersuchung der biologischen Zellen 14 in dem Zellgefäß 1, die dazu geeignet ist, von biologischen Zellen 14 aus dem Zellgefäß 1 emittierte elektromagnetische Strahlung zu detektieren, ist in dieser Ausgestaltungsform ein Fluoreszenzmikroskop.

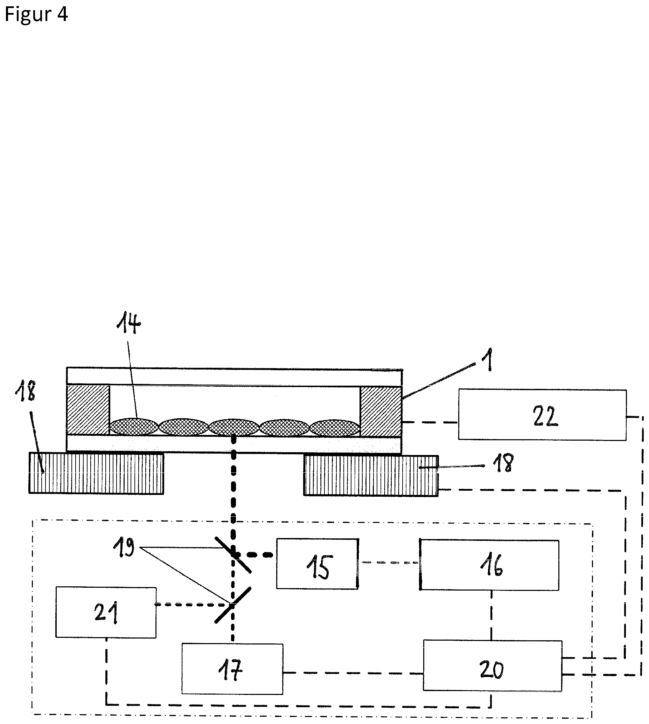

Figur 4 zeigt eine weitere Ausgestaltungsform einer erfindungsgemäßen Vorrichtung, die auf einem x,y,z-Positioniersystem 18 angeordnet ist. In dieser Ausgestaltungsform wird elektromagnetische Strahlung des Geräts 15 zur Schädigung biologischer Zellen (hier: ein Laser) über einen Spiegel 19 (bzw. Strahlteiler) auf die biologischen Zellen 14 in dem Zellgefäß 1 gelenkt. Der Laser 15 ist auch hier kommunikativ mit einer Steuereinheit 16 zur Steuerung des Lasers verbunden, welche wiederrum über eine Systemsteuerung 20 gesteuert wird. Die Systemsteuerung 20 ist zudem kommunikativ mit der Vorrichtung 17 zur Untersuchung der biologischen Zellen 14 in dem Zellgefäß 1 verbunden (hier: eine Kamera). Die Systemsteuerung 20 ist hier ferner mit einer zusätzlichen Lichtquelle 21 verbunden, mit der zusätzliches (z.B. für die biologischen Zellen 14 unschädliches) Licht auf die biologischen Zellen 14 abgelenkt werden kann. Die Ablenkung des Lichts der zusätzlichen Lichtquelle 21 geschieht in dieser Ausführungsform ebenfalls über den Spiegel 19 (bzw. Strahlteiler). Die Systemsteuerung ist hier zudem kommunikativ mit einer Steuerung 22 verbunden, mit welcher die Vorrichtung zur Bewegung einer Flüssigkeit in dem Zellgefäß 1 (z.B. eine Mikropumpe) gesteuert werden kann.

## Patentansprüche

1. Vorrichtung für Zellkulturexperimente, enthaltend
a) mindestens ein Zellgefäß zur Kultivierung von biologischen Zellen;
b) ein Gerät zur Schädigung biologischer Zellen, wobei das Gerät dazu geeignet ist, elektromagnetische Strahlung auf biologische Zellen in dem Zellgefäß zu emittieren;
c) eine Steuereinheit zur Steuerung des Geräts zur Schädigung biologischer Zellen in dem Zellgefäß; und
d) eine Vorrichtung zur Untersuchung der biologischen Zellen in dem Zellgefäß, wobei die Vorrichtung dazu geeignet ist, von biologischen Zellen aus dem Zellgefäß emittierte elektromagnetische Strahlung zu detektieren,
**dadurch gekennzeichnet, dass**
die Vorrichtung eine mikrofluidische Vorrichtung ist,
die mikrofluidische Vorrichtung eine Mikropumpe zur Bewegung einer Flüssigkeit in dem Zellgefäß enthält,
die mikrofluidische Vorrichtung aus mehreren, übereinander angeordneten Schichten aufgebaut ist, wobei mindestens eine Schicht ein pneumatisches Subsystem aufweist, das mindestens einen Kanal zum Transport eines Gases enthält,
das Zellgefäß in einem fluidischen Kreislauf angeordnet ist, und
die mikrofluidische Vorrichtung eine Systemsteuervorrichtung enthält, wobei die Systemsteuervorrichtung dazu konfiguriert ist, die Steuereinheit zur Steuerung des Geräts zur Schädigung biologischer Zellen, die Vorrichtung zur Untersuchung der biologischen Zellen und die Mikropumpe zur Bewegung einer Flüssigkeit zu steuern.

2. Mikrofluidische Vorrichtung gemäß Anspruch 1, **dadurch gekennzeichnet, dass** die mikrofluidische Vorrichtung, bevorzugt das Zellgefäß zur Kultivierung biologischer Zellen
i) mindestens eine Kammer aufweist, wobei die mindestens eine Kammer bevorzugt eine Länge, Breite und/oder Höhe unter 1000 µm, besonders bevorzugt eine Länge, Breite und/oder Höhe von 1 µm bis 1000 µm, insbesondere eine Länge, Breite und Höhe im Bereich von 1 µm bis 1000 µm hat; und/oder
ii) mindestens einen Kanal aufweist, wobei der mindestens eine Kanal senkrecht zur Ausbreitungsrichtung des Kanals, bevorzugt eine Ausdehnung von maximal 1000 µm, besonders bevorzugt eine Ausdehung von 1 µm bis 1000 µm hat; und/oder
iii) mindestens eine Oberfläche mit einer Markierung aufweist, bevorzugt mindestens eine Oberfläche mit einem Gitter, besonders bevorzugt mindestens eine Oberfläche mit einem mit Koordinaten versehenen Gitter, wobei die Oberfläche insbesondere eine Bodenfläche des Zellgefäßes ist; und/oder
iv) fluidisch mit einer Mikropumpe verbunden ist, wobei die Mikropumpe bevorzugt mindestens ein Einlassventil, mindestens eine Pumpkammer und mindestens ein Auslassventil enthält oder daraus besteht; und/oder
v) symmetrisch aufgebaut ist, wobei die mikrofluidische Vorrichtung bevorzugt mindestens zwei symmetrisch angeordnete fluidische Kreisläufe enthält.

3. Mikrofluidische Vorrichtung gemäß einem der vorhergehenden Ansprüche, **dadurch gekennzeichnet, dass**
i) das pneumatische Subsystem mehrere Kanäle zum Transport eines Gases enthält; und/oder
ii) die Vorrichtung mindestens eine selektiv permeable Schicht enthält, die für Gas, bevorzugt für Sauerstoff und/oder Kohlendioxid, permeabel ist und/oder für Flüssigkeit impermeabel ist, wobei die selektiv permeable Schicht bevorzugt eine elastomere Membran enthält oder daraus besteht; und/oder
iii) die Vorrichtung mindestens eine Schicht, bevorzugt mehrere Schichten, ein fluidisches Subsystem aufweist, wobei das fluidische Subsystem insbesondere mindestens eine Schicht mit mindestens einer Mikropumpe, mindestens eine Schicht mit mindestens einem Zellgefäß, mindestens eine Schicht mit Kanälen und/oder mindestens eine Schicht mit Markierungen enthält.

4. Mikrofluidische Vorrichtung gemäß einem der vorhergehenden Ansprüche, **dadurch gekennzeichnet, dass** die mikrofluidische Vorrichtung, bevorzugt das Zellgefäß zur Kultivierung biologischer Zellen,
i) eine Quelle ausgewählt aus der Gruppe bestehend aus Heizquelle, Kühlquelle, Gasquelle, Flüssigkeitsquelle und Kombinationen hiervon, bevorzugt eine Quelle ausgewählt aus der Gruppe bestehend aus Heizquelle zur Temperierung auf 25 bis 37 °C, Sauerstoffquelle, Kohlendioxidquelle, Wasserdampfquelle und Kombinationen hiervon, enthält; und/oder
ii) ein Material ausgewählt aus der Gruppe bestehend aus Polymere, bevorzugt ein Material ausgewählt aus der Gruppe bestehend aus Polycarbonat, Cyclo-Olefin-Copolymere, transparente Elastomere, Glas, und Mischungen hiervon, besonders ein Material ausgewählt aus der Gruppe bestehend aus PDMS, Topas^{®}-Cyclo-Olefin-Copolymer und Mischungen hiervon, enthält oder daraus besteht.

5. Mikrofluidische Vorrichtung gemäß einem der vorhergehenden Ansprüche, **dadurch gekennzeichnet, dass** die Mikropumpe mindestens ein Einlassventil, mindestens eine Pumpkammer und mindestens ein Auslassventil enthält oder daraus besteht.

6. Mikrofluidische Vorrichtung gemäß einem der vorhergehenden Ansprüche, **dadurch gekennzeichnet, dass** die mikrofluidische Vorrichtung, bevorzugt das Gerät zur Schädigung biologischer Zellen, eine elektromagnetische Strahlungsquelle enthält, bevorzugt eine elektromagnetische Strahlungsquelle, die dazu geeignet ist
i) elektromagnetische Strahlung im UV-Bereich und/oder VIS-Bereich, abzustrahlen; und/oder
ii) kohärente und/oder monochromatische elektromagnetische Strahlung abzustrahlen, wobei das Gerät bevorzugt einen Laser enthält oder daraus besteht.

7. Mikrofluidische Vorrichtung gemäß einem der vorhergehenden Ansprüche, **dadurch gekennzeichnet, dass** die mikrofluidische Vorrichtung eine Schneidevorrichtung enthält, bevorzugt eine Schneidevorrichtung zum Schneiden von dem Zellgefäß zur Kultivierung biologischer Zellen, besonders bevorzugt eine Schneidvorrichtung zum Schneiden von einer Kammer, einem Kanal und/oder einer Zellwand der mikrofluidischen Vorrichtung.

8. Mikrofluidische Vorrichtung gemäß einem der vorhergehenden Ansprüche, **dadurch gekennzeichnet, dass** die Steuervorrichtung dazu konfiguriert ist, das Gerät zur Schädigung biologischer Zellen über eine Steuergröße ausgewählt aus der Gruppe bestehend aus Bestrahlungsposition entlang des Zellgefäßes, Bestrahlungsbeginn, Bestrahlungsende, Bestrahlungszeit und Bestrahlungsintensität zu steuern.

9. Mikrofluidische Vorrichtung gemäß einem der vorhergehenden Ansprüche, **dadurch gekennzeichnet, dass** die mikrofluidische Vorrichtung, bevorzugt das Gerät zur Schädigung biologischer Zellen und/oder die Vorrichtung zur Untersuchung biologischer Zellen,
i) eine optische Strahlenführung zur Leitung elektromagnetischer Strahlung, bevorzugt eine optische Strahlenführung ausgewählt aus der Gruppe bestehend aus optische Faser, optischer Wellenleiter, digitaler Mikrospiegel und Kombinationen hiervon; und/oder
ii) eine Vorrichtung zur Fokussierung elektromagnetischer Strahlung, bevorzugt eine Vorrichtung ausgewählt aus der Gruppe bestehend aus Linse, Spiegel und Kombinationen hiervon; und/oder
iii) eine Vorrichtung zur Filterung elektromagnetischer Strahlung, bevorzugt ausgewählt aus der Gruppe bestehend aus optischer Filter, digitaler Mikrospiegel und Kombinationen hiervor, besonders bevorzugt einen optischen, dichroitischen Filter, einen digitalen Mikrospiegel und Kombinationen hiervon;
enthält.

10. Mikrofluidische Vorrichtung gemäß einem der vorhergehenden Ansprüche, **dadurch gekennzeichnet, dass** die mikrofluidische Vorrichtung, bevorzugt die Vorrichtung zur Untersuchung biologischer Zellen,
i) ein optisches Mikroskop, bevorzugt ein optisches und elektronisches Mikroskop, enthält oder daraus besteht; und/oder
ii) elektromagnetische Strahlungsquelle, bevorzugt eine elektromagnetische Strahlungsquelle mit homogener Intensitätsverteilung, wobei die Strahlungsquelle insbesondere die dazu geeignet ist, elektromagnetische Strahlung im UV-Bereich und/oder VIS-Bereich abzustrahlen; und/oder
iii) eine Detektionsvorrichtung zur Detektion von elektromagnetischer Strahlung, bevorzugt einen HSI-Detektor, besonders bevorzugt einen HSI-Detektor zur Detektion von elektromagnetischer Strahlung im UV-Bereich, VIS-Bereich, IR-Bereich und/oder FIR-Bereich, insbesondere einen HSI-Detektor zur Detektion von elektromagnetischer Strahlung im UV-Bereich, VIS-Bereich, IR-Bereich und FIR-Bereich;
enthält.

11. Mikrofluidische Vorrichtung gemäß einem der vorhergehenden Ansprüche, **dadurch gekennzeichnet, dass** die mikrofluidische Vorrichtung, bevorzugt die Vorrichtung zur Untersuchung biologischer Zellen, eine Aufzeichnungsvorrichtung, bevorzugt eine Kamera, besonders bevorzugt einer Kamera zur Detektion von elektromagnetischer Strahlung im Nah-IR und/oder VIS-Bereich, besonders bevorzugt eine CCD-Kamera, enthält.

12. Mikrofluidische Vorrichtung gemäß einem der vorhergehenden Ansprüche, **dadurch gekennzeichnet, dass** die mikrofluidische Vorrichtung, bevorzugt die Vorrichtung zur Untersuchung biologischer Zellen und/oder das Gerät zur Schädigung biologischer Zellen, ein Ortungssystem, bevorzugt ein Ortungssystem zur Verfolgung von Zellen, besonders bevorzugt ein Ortungssystem zur Verfolgung einzelner Zellen, insbesondere eine Kombination aus Kamera und Bildauswertungsprogramm, enthält.

13. Mikrofluidische Vorrichtung gemäß einem der vorhergehenden Ansprüche, **dadurch gekennzeichnet, dass** die Vorrichtung zur Untersuchung biologischer Zellen optisch und/oder elektronisch an das Gerät zur Schädigung biologischer Zellen gekoppelt ist, wobei die Gerät zur Schädigung biologischer Zellen und die Vorrichtung zur Untersuchung biologischer Zellen optional als eine zusammenhängende Einheit ausgestaltet sind.

14. Mikrofluidische Vorrichtung gemäß einem der vorhergehenden Ansprüche, **dadurch gekennzeichnet, dass** die Systemsteuervorrichtung zudem dazu konfiguriert ist, eine zusätzliche Lichtquelle zu steuern.

15. Verwendung einer mikrofluidischen Vorrichtung gemäß einem der vorhergehenden Ansprüche zur
i) Untersuchung einer Schädigung von biologischen Zellen, bevorzugt zur Schädigung der zellulären Membran von biologischen Zellen, besonders bevorzugt zur Untersuchung der Wirkung von mindestens einem Medikament auf die Schädigung der zellulären Membran von biologischen Zellen; und/oder
ii) Untersuchung einer Regeneration von biologischen Zellen, bevorzugt zur Untersuchung der Regeneration von biologischen Zellen nach einer Schädigung, besonders bevorzugt zur Untersuchung der Wirkung von mindestens einem Medikament auf die Regeneration von biologischen Zellen nach einer Schädigung; und/oder
iii) Untersuchung der räumlichen Interaktion verschiedener Zelltypen, bevorzugt der räumlichen Interaktion von immunologischen Zelltypen mit Endothelzellen.

## Claims

1. A device for cell culture experiments, comprising
a) at least one cell vessel for cultivating biological cells;
b) an apparatus for damaging biological cells, the apparatus being suitable for emitting electromagnetic radiation onto biological cells in the cell vessel;
c) a control unit for controlling the apparatus for damaging biological cells in the cell vessel; and
d) a device for examining the biological cells in the cell vessel, the device being suitable for detecting electromagnetic radiation emitted by biological cells from the cell vessel,
**characterized in that**
the device is a microfluidic device,
the microfluidic device comprises a micropump for moving a liquid in the cell vessel,
the microfluidic device is constructed from a plurality of layers arranged one above the other, at least one layer comprising a pneumatic sub-system which comprises at least one channel for transporting a gas,
the cell vessel is arranged in a fluidic circuit, and
the microfluidic device comprises a system control device, the system control device being configured to control the control unit for controlling the apparatus for damaging biological cells, the device for examining the biological cells, and the micropump for moving a liquid.

2. The microfluidic device according to claim 1, **characterized in that** the microfluidic device, preferably the cell vessel for cultivating biological cells,
i) comprises at least one chamber, the at least one chamber preferably having a length, width, and/or height below 1000 µm, particularly preferably a length, width, and/or height of from 1 µm to 1000 µm, in particular a length, width, and height in the range of from 1 µm to 1000 µm; and/or
ii) comprises at least one channel, the at least one channel preferably having an extent of at most 1000 µm, particularly preferably an extent of 1 µm to 1000 µm, perpendicularly to the projection direction of the channel; and/or
iii) comprises at least one surface having a marker, preferably at least one surface having a grid, particularly preferably at least one surface having a grid provided with coordinates, the surface in particular being a bottom surface of the cell vessel; and/or
iv) is fluidically connected to a micropump, the micropump preferably comprising or consisting of at least one inlet valve, at least one pump chamber, and at least one outlet valve; and/or
v) is constructed symmetrically, the microfluidic device preferably comprising at least two symmetrically arranged fluidic circuits.

3. The microfluidic device according to any one of the preceding claims, **characterized in that**
i) the pneumatic sub-system comprises a plurality of channels for transporting a gas; and/or
ii) the device comprises at least one selectively permeable layer, which is permeable to gas, preferably to oxygen and/or carbon dioxide, and/or is impermeable to liquid, the selectively permeable layer preferably comprising or consisting of an elastomer membrane; and/or
iii) the device comprises at least one layer, preferably a plurality of layers, and a fluidic sub-system, the fluidic sub-system in particular comprising at least one layer comprising at least one micropump, at least one layer comprising at least one cell vessel, at least one layer comprising channels, and/or at least one layer comprising markers.

4. The microfluidic device according to any one of the preceding claims, **characterized in that** the microfluidic device, preferably the cell vessel for cultivating biological cells,
i) comprises a source selected from the group consisting of a heating source, a cooling source, a gas source, a liquid source, and combinations thereof, preferably a source selected from the group consisting of a heating source for controlling the temperature to 25 to 37°C, an oxygen source, a carbon dioxide source, a steam source, and combinations thereof; and/or
ii) comprises or consists of a material selected from the group consisting of polymers, preferably a material selected from the group consisting of polycarbonate, cyclic olefin copolymers, transparent elastomers, glass, and mixtures thereof, particularly a material selected from the group consisting of PDMS, TOPAS^{®} cyclic olefin copolymers, and mixtures thereof.

5. The microfluidic device according to any one of the preceding claims, **characterized in that** the micropump comprises or consists of at least one inlet valve, at least one pump chamber, and at least one outlet valve.

6. The microfluidic device according to any one of the preceding claims, **characterized in that** the microfluidic device, preferably the apparatus for damaging biological cells, comprises an electromagnetic radiation source, preferably an electromagnetic radiation source which is suitable for
i) emitting electromagnetic radiation in the UV range and/or in the VIS range; and/or
ii) emitting coherent and/or monochromatic electromagnetic radiation, the apparatus preferably comprising or consisting of a laser.

7. The microfluidic device according to any one of the preceding claims, **characterized in that** the microfluidic device comprises a cutting device, preferably a cutting device for cutting the cell vessel for cultivating biological cells, particularly preferably a cutting device for cutting a chamber, a channel, and/or a cell wall of the microfluidic device.

8. The microfluidic device according to any one of the preceding claims, **characterized in that** the control device is configured to control the apparatus for damaging biological cells by means of a control variable selected from the group consisting of an irradiation position along the cell vessel, a start of irradiation, an end of irradiation, an irradiation time, and an irradiation intensity.

9. The microfluidic device according to any one of the preceding claims, **characterized in that** the microfluidic device, preferably the apparatus for damaging biological cells and/or the device for examining biological cells, comprises
i) an optical beam guide for conducting electromagnetic radiation, preferably an optical beam guide selected from the group consisting of optical fibers, optical waveguides, digital micromirrors, and combinations thereof; and/or
ii) a device for focusing electromagnetic radiation, preferably a device selected from the group consisting of lens, mirror, and combinations thereof; and/or
iii) a device for filtering electromagnetic radiation, preferably selected from the group consisting of optical filter, digital micromirror, and combinations thereof, particularly preferably an optical dichroic filter, a digital micromirror, and combinations thereof.

10. The microfluidic device according to any one of the preceding claims, **characterized in that** the microfluidic device, preferably the device for examining biological cells,
i) comprises or consists of an optical microscope, preferably an optical and electronic microscope; and/or
ii) comprises an electromagnetic radiation source, preferably an electromagnetic radiation source having a homogeneous intensity distribution, the radiation source in particular being suitable for emitting electromagnetic radiation in the UV range and/or in the VIS range; and/or
iii) comprises a detection device for detecting electromagnetic radiation, preferably an HSI detector, particularly preferably an HSI detector for detecting electromagnetic radiation in the UV range, the VIS range, the IR range, and/or the FIR range, in particular an HSI detector for detecting electromagnetic radiation in the UV range, the VIS range, the IR range, and the FIR range.

11. The microfluidic device according to any one of the preceding claims, **characterized in that** the microfluidic device, preferably the device for examining biological cells, comprises a recording device, preferably a camera, particularly preferably a camera for detecting electromagnetic radiation in the near IR and/or VIS range, particularly preferably a CCD camera.

12. The microfluidic device according to any one of the preceding claims, **characterized in that** the microfluidic device, preferably the device for examining biological cells and/or the apparatus for damaging biological cells, comprises a positioning system, preferably a positioning system for tracking cells, particularly preferably a positioning system for tracking individual cells, in particular a combination of a camera and an image analysis program.

13. The microfluidic device according to any one of the preceding claims, **characterized in that** the device for examining biological cells is optically and/or electronically coupled to the apparatus for damaging biological cells, the apparatus for damaging biological cells and the device for examining biological cells optionally being configured as one cohesive unit.

14. The microfluidic device according to any one of the preceding claims, **characterized in that** the system control device is also configured to control an additional light source.

15. A use of a microfluidic device according to any one of the preceding claims for
i) examining a damage to biological cells, preferably for damaging the cellular membrane of biological cells, particularly preferably for examining an effect of at least one drug on the damage to the cellular membrane of biological cells; and/or
ii) examining a regeneration of biological cells, preferably for examining a regeneration of biological cells following damage, particularly preferably for examining an effect of at least one drug on the regeneration of biological cells following damage; and/or
iii) examining a spatial interaction of different cell types, preferably a spatial interaction of immunological cell types comprising endothelial cells.

## Revendications

1. Dispositif pour expériences de culture cellulaire, contenant
a) au moins un récipient de culture cellulaire permettant de cultiver des cellules biologiques ;
b) un appareil permettant d'endommager des cellules biologiques, dans lequel ledit appareil est approprié pour émettre un rayonnement électromagnétique sur des cellules biologiques au sein du récipient de culture cellulaire ;
c) une unité de commande permettant de commander l'appareil permettant d'endommager des cellules biologiques dans le récipient de culture cellulaire ; et
d) un dispositif permettant d'examiner les cellules biologiques dans le récipient de culture cellulaire, dans lequel le dispositif est approprié pour détecter le rayonnement électromagnétique émis par des cellules biologiques depuis le récipient de culture cellulaire,
**caractérisé en ce que**
le dispositif est un dispositif microfluidique,
le dispositif microfluidique contient une micropompe permettant de déplacer un fluide au sein du récipient de culture cellulaire,
le dispositif microfluidique est composé de plusieurs couches agencées les unes au-dessus des autres, dans lequel au moins une couche présente un sous-système pneumatique contenant au moins un canal permettant le transport d'un gaz,
le récipient de culture cellulaire est agencé selon un circuit fluidique, et
le dispositif microfluidique contient un dispositif de commande de système, dans lequel le dispositif de commande de système est configuré pour commander l'unité de commande permettant de commander l'appareil permettant d'endommager des cellules biologiques, le dispositif permettant d'examiner les cellules biologiques et la micropompe permettant de déplacer un fluide.

2. Dispositif microfluidique selon la revendication 1, **caractérisé en ce que** le dispositif microfluidique, de manière préférée le récipient de culture cellulaire permettant de cultiver des cellules biologiques,
i) présente au moins une chambre, dans lequel la au moins une chambre présente de manière préférée une longueur, une largeur et/ou une hauteur inférieures à 1000 µm, de manière particulièrement préférée une longueur, une largeur et/ou une hauteur comprises entre 1 µm et 1000 µm, en particulier une longueur, une largeur et/ou une hauteur situées dans la plage comprise entre 1 µm et 1000 µm ; et/ou
ii) présente au moins un canal, dans lequel le au moins un canal présente, perpendiculairement à la direction de propagation du canal, de manière préférée une extension maximale de 1000 µm, de manière particulièrement préférée une extension comprise entre 1 µm et 1000 µm ; et/ou
iii) présente au moins une surface présentant un marquage, de manière préférée au moins une surface présentant une grille, de manière particulièrement préférée au moins une surface présentant une grille munie de coordonnées, dans lequel la surface est en particulier une surface inférieure du récipient de culture cellulaire ; et/ou
iv) est relié fluidiquement à une micropompe, dans lequel la micropompe contient ou est constituée de manière préférée d'au moins une soupape d'entrée, d'au moins une chambre de pompage et d'au moins une soupape de sortie ; et/ou
v) est de construction symétrique, dans lequel le dispositif microfluidique contient de manière préférée au moins deux circuits fluidiques agencés symétriquement.

3. Dispositif microfluidique selon l'une quelconque des revendications précédentes, **caractérisé en ce que**
i) le sous-système pneumatique contient plusieurs canaux permettant le transport d'un gaz ; et/ou
ii) le dispositif contient de manière préférée au moins une couche sélectivement perméable qui est perméable aux gaz, de manière préférée à l'oxygène et/ou au dioxyde de carbone, et/ou qui est imperméable aux liquides, dans lequel la couche sélectivement perméable contient de manière préférée une membrane élastomère ou est constituée de celle-ci ; et/ou
iii) le dispositif présente au moins une couche, de manière préférée plusieurs couches, d'un sous-système fluidique, dans lequel le sous-système fluidique contient en particulier au moins une couche avec au moins une micropompe, au moins une couche avec au moins un récipient de culture cellulaire, au moins une couche avec des canaux et/ou au moins une couche avec des marquages.

4. Dispositif microfluidique selon l'une quelconque des revendications précédentes, **caractérisé en ce que** le dispositif microfluidique, de manière préférée le récipient de culture cellulaire permettant de cultiver des cellules biologiques,
i) contient une source choisie parmi le groupe constitué d'une source de chauffage, d'une source de refroidissement, d'une source de gaz, d'une source de liquide et de leurs combinaisons, de manière préférée une source choisie parmi le groupe constitué d'une source de chauffage permettant une mise en température entre 25 et 37°C, d'une source d'oxygène, d'une source de dioxyde de carbone, d'une source de vapeur d'eau et de leurs combinaisons ; et/ou
ii) contient, ou est constitué de, un matériau choisi parmi le groupe des polymères, de manière préférée un matériau choisi parmi le groupe constitué du polycarbonate, des copolymères de cyclo-oléfine, d'élastomères transparents, de verre et de leurs mélanges, en particulier un matériau choisi parmi le groupe constitué du PDMS, du copolymère de cyclo-oléfine Topas^{®} et de leurs mélanges.

5. Dispositif microfluidique selon l'une quelconque des revendications précédentes, **caractérisé en ce que** la micropompe contient, ou est constituée de, au moins une soupape d'entrée, au moins une chambre de pompage et au moins une soupape de sortie.

6. Dispositif microfluidique selon l'une quelconque des revendications précédentes, **caractérisé en ce que** le dispositif microfluidique, de manière préférée l'appareil permettant d'endommager des cellules biologiques, contient une source de rayonnement électromagnétique, de manière préférée une source de rayonnement électromagnétique, qui est appropriée pour
i) émettre un rayonnement électromagnétique dans le domaine UV et/ou le domaine VIS ; et/ou
ii) émettre un rayonnement électromagnétique cohérent et/ou monochromatique, dans lequel le dispositif contient de manière préférée un laser ou est constitué de celui-ci.

7. Dispositif microfluidique selon l'une quelconque des revendications précédentes, **caractérisé en ce que** le dispositif microfluidique contient un dispositif de découpe, de manière préférée un dispositif de découpe permettant de découper le récipient de culture cellulaire permettant de cultiver des cellules biologiques, et de manière particulièrement préférée un dispositif de découpe permettant de découper une chambre, un canal et/ou une paroi de cellule du dispositif microfluidique.

8. Dispositif microfluidique selon l'une quelconque des revendications précédentes, **caractérisé en ce que** le dispositif de commande est configuré pour commander l'appareil permettant d'endommager des cellules biologiques, par l'intermédiaire d'une variable de commande choisie parmi le groupe constitué de la position d'irradiation le long du récipient de culture cellulaire, du début d'irradiation, de la fin d'irradiation, du temps d'irradiation et de l'intensité d'irradiation.

9. Dispositif microfluidique selon l'une quelconque des revendications précédentes, **caractérisé en ce que** le dispositif microfluidique, de manière préférée l'appareil permettant d'endommager des cellules biologiques et/ou le dispositif permettant d'examiner des cellules biologiques, contient
i) un guide de faisceau optique permettant de diriger le rayonnement électromagnétique, de manière préférée un guide de faisceau optique choisi parmi le groupe constitué des fibres optiques, des guides d'ondes optiques, des micromiroirs numériques et de leurs combinaisons ; et/ou
ii) un dispositif permettant de focaliser le rayonnement électromagnétique, de manière préférée un dispositif choisi parmi le groupe constitué d'une lentille, d'un miroir et de leurs combinaisons ; et/ou
iii) un dispositif permettant de filtrer le rayonnement électromagnétique, de manière préférée choisi parmi le groupe constitué des filtres optiques, des micromiroirs numériques et de leurs combinaisons, de manière particulièrement préférée d'un filtre dichroïque optique, d'un micromiroir numérique et de leurs combinaisons.

10. Dispositif microfluidique selon l'une quelconque des revendications précédentes, **caractérisé en ce que** le dispositif microfluidique, de manière préférée le dispositif permettant d'examiner des cellules biologiques,
i) contient, ou est constitué de, un microscope optique, de manière préférée un microscope optique et électronique ; et/ou
ii) contient une source de rayonnement électromagnétique, de manière préférée une source de rayonnement électromagnétique à distribution d'intensité homogène, dans lequel la source de rayonnement est en particulier appropriée pour émettre un rayonnement électromagnétique dans le domaine UV et/ou le domaine VIS ; et/ou
iii) contient un dispositif de détection permettant de détecter un rayonnement électromagnétique, de manière préférée un détecteur HSI, de manière particulièrement préférée un détecteur HSI permettant de détecter un rayonnement électromagnétique dans le domaine UV, le domaine VIS, le domaine IR et/ou le domaine FIR, en particulier un détecteur HSI permettant de détecter un rayonnement électromagnétique dans le domaine UV, le domaine VIS, le domaine IR et le domaine FIR.

11. Dispositif microfluidique selon l'une quelconque des revendications précédentes, **caractérisé en ce que** le dispositif microfluidique, de manière préférée le dispositif permettant d'examiner des cellules biologiques, contient un dispositif d'enregistrement, de manière préférée une caméra, de manière particulièrement préférée une caméra permettant de détecter un rayonnement électromagnétique dans le domaine du proche infrarouge et/ou dans le domaine visible, de manière particulièrement préférée une caméra CCD.

12. Dispositif microfluidique selon l'une quelconque des revendications précédentes, **caractérisé en ce que** le dispositif microfluidique, de manière préférée le dispositif permettant d'examiner des cellules biologiques et/ou l'appareil permettant d'endommager des cellules biologiques, contient un système de localisation, de manière préférée un système de localisation permettant un suivi des cellules, de manière particulièrement préférée un système de localisation permettant un suivi de cellules individuelles, en particulier une combinaison d'une caméra et d'un programme d'évaluation d'image.

13. Dispositif microfluidique selon l'une quelconque des revendications précédentes, **caractérisé en ce que** le dispositif permettant d'examiner des cellules biologiques est couplé optiquement et/ou électroniquement à l'appareil permettant d'endommager des cellules biologiques, dans lequel l'appareil permettant d'endommager des cellules biologiques et le dispositif permettant d'examiner des cellules biologiques sont éventuellement réalisés sous la forme d'une unité intégrée.

14. Dispositif microfluidique selon l'une quelconque des revendications précédentes, **caractérisé en ce que** le dispositif de commande de système est en outre configuré pour commander une source lumineuse supplémentaire.

15. Utilisation d'un dispositif microfluidique selon l'une quelconque des revendications précédentes pour
i) examiner un dommage causé à des cellules biologiques, de manière préférée pour endommager la membrane cellulaire de cellules biologiques, de manière particulièrement préférée pour examiner l'effet d'au moins un médicament sur la lésion de la membrane cellulaire de cellules biologiques ; et/ou
ii) examiner la régénération des cellules biologiques, de manière préférée pour examiner la régénération de cellules biologiques après une lésion, de manière particulièrement préférée pour examiner l'effet d'au moins un médicament sur la régénération de cellules biologiques après une lésion ; et/ou
iii) examiner l'interaction spatiale de différents types cellulaires, de manière préférée l'interaction spatiale de types cellulaires immunologiques avec des cellules endothéliales.
